(19) Europäisches Patentamt

European Patent Office

Office européen des brevets

(11) **EP 1 061 370 A2**

(12) **EUROPEAN PATENT APPLICATION**

(43) Date of publication:
**20.12.2000 Bulletin 2000/51**

(51) Int. Cl.⁷: **G01N 33/574**

(21) Application number: **00112207.6**

(22) Date of filing: **07.06.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **18.06.1999 JP 17248599**

(71) Applicant:
**Wako Pure Chemical Industies, Ltd.
Osaka 540-8605 (JP)**

(72) Inventors:
• **Hosokawa, Hideaki
Amagasaki-shi, Hyogo 661-0963 (JP)**
• **Nakamura, Kenji
Amagasaki-shi, Hyogo 661-0963 (JP)**
• **Satomura, Shinji
Osaka 540-8605 (JP)**

(74) Representative:
**von Kreisler, Alek, Dipl.-Chem. et al
Patentanwälte,
von Kreisler-Selting-Werner,
Bahnhofsvorplatz 1 (Deichmannhaus)
50667 Köln (DE)**

(54) **A method for detection of carcinoembryonic antigens having a modified sugar chain structure**

(57)     The present invention relates to a method for detection of carcinoembryonic antigens (hereinafter abbreviated as CEAs) having a modified sugar chain structure which comprises using an antibody against a constant region of CEAs and a protein capable of recognizing a modified sugar chain structure of CEAs, a method for detecting a cancer which comprises using an amount of CEAs having a modified sugar chain structure and a kit for detection of CEAs having a modified sugar chain structure which comprises an antibody against a constant region of CEAs and a protein capable of recognizing a modified sugar chain structure of CEAs.

    Said method and kit are useful in a separately measurement of the various CEAs, a detection of a cancer and a definition of the kind of a cancer, and, for example, by using in proper combination of the measurement value of various CEAs obtained by the method of the present invention, it becomes possible to detect the cancer or define the kind of the cancer.

EP 1 061 370 A2

# EP 1 061 370 A2

## Description

### BACKGROUND OF INVENTION

The present invention relates to a method for detection of carcinoembryonic antigens having various sugar chains (i.e. a normal type sugar chain and various modified sugar chains) (hereinafter abbreviated as CEAs), in particular, a method and kit for detection of CEAs having a modified sugar chain structure.

The present invention also relates to a method for detection of a cancer which comprises measuring an amount of CEAs having a modified sugar chain structure and detecting cancer on the basis of this amount.

So-called carcinoembryonic antigen (hereinafter abbreviated as CEA) is one of embryonic antigens originated from cancer cells, which is composed of a glycoprotein having a molecular weight of about 200,000 and a sugar content of about 50 %. CEA is produced from a surface of a normal mucosal cell of digestive organs and also from tumor tissues. It has been known that no CEA is found in blood of normal human beings but an amount of CEA is increased in blood and cells of human being suffering from organ cancers such as colorectal cancer, lung cancer, stomach cancer, breast cancer and liver cancer. Therefore, an amount of CEA has been useful as a marker for detecting various kinds of cancers, and measurement of an amount of CEA in blood has widely been used for screening of cancers, observation of progress of diseases after operation and prevention of recurrence of cancers. However, detection of cancers has been difficult to conduct, if the cancers are at rather early stage, only on the basis of the amount of CEA in blood.

Therefore, for the purpose of detection of cancer cells at rather early stage, an X-ray diagnosis, an endoscopy diagnosis etc. have been conducted. However, there have been such problems that detection of cancers at rather early stage is hardly possible by an X-ray diagnosis, and an endoscopy diagnosis is influenced by a level of a diagnosis technique and knowledge.

On the other hand, CEAs from normal mucosal cells of digestive organs capable of producing CEAs, and those from tumor cells have been obtained separately in pure state, and analytical studies have been conducted on the sugar chain structures of those CEAs, whereby there has been found a difference in the sugar chain structures between CEAs from normal mucosal cells of digestive organs and those from cancer cells. And consequently use of those phenomena has been expected as useful for cancer diagnosis. (Katsuko Yamashita, J. Biol. Chem., 264 (30), 17873-17881(1989), Katsuko Yamashita, Glycobiology, 5 (1), 105-115 (1995)) Possible methods using this phenomena, however, would involve such problems that purification of CEAs and analysis of sugar chain structures thereof are necessarily required and therefore it takes a lot of time to conduct the necessary diagnosis.

### SUMMARY OF INVENTION

The present invention has been accomplished taking the above mentioned situation into consideration, and its object is to provide an easy and simple method for detection of CEAs in samples originated from a living body, to provide a method for detection of cancers on the basis of the CEAs amounts or, in other words, a method for detection of cancer by using the amounts as an indicator and also to provide a kit used in said methods.

Namely, the present inventors have earnestly investigated in order to solve the above mentioned theme to reach such a finding that the total amount of CEAs in a sample originated from a living body, an amount of CEAs having a specific modified sugar chain structure and/or an amount of CEAs having a sugar chain structure other than the specific one can be detected by using an antibody against a constant region of CEAs and a protein capable of recognizing a modified sugar chain structure of CEAs. And present inventors have further investigated to arrive at the finding that an amount of CEAs having the specific modified sugar chain structure or an amount of CEAs having a sugar chain structure other than the specific one, or a ratio of an amount of the CEAs having the specific modified sugar chain structure or a ratio of an amount of the CEAs having a sugar chain structure other than the specific one relative to the total amount of CEAs is useful, for example, for detection of colorectal cancer. The present invention has been accomplished on the basis of these findings.

The present invention comprises the following features in order to attain the above-mentioned object.

(1) A method for detection of CEAs having a modified sugar chain structure which comprises using an antibody against a constant region of CEAs (hereinafter abbreviated as a CEAs binding antibody) and a protein capable of recognizing a modified sugar chain structure of CEAs (hereinafter abbreviated as a specific sugar chain binding protein).
(2) A method for detection of CEAs having a modified sugar chain structure which comprises measuring an amount of a complex of CEAs, a CEAs binding antibody and a specific sugar chain binding protein.
(3) A method for detection of CEAs having a modified sugar chain structure which comprises

reacting a sample with a CEAs binding antibody and a specific sugar chain binding protein to give a complex of CEAs, the CEAs binding antibody and the specific sugar chain binding protein, and

measuring an amount of the complex.

(4) A method for detecting a cancer which comprises using an amount of a CEAs having a modified sugar chain structure as an indicator for the detection.

(5) A method for detecting a cancer which comprises

measuring an amount of a complex of CEAs, a CEAs binding antibody and a specific sugar chain binding protein, and

using the amount as an indicator for the detection.

(6) A method for detecting a cancer which comprises

measuring an amount of a complex of CEAs, a CEAs binding antibody and a specific sugar chain binding protein, and an amount of a complex of CEAs and the specific sugar chain binding protein, and

using the amounts as an indicator for the detection.

(7) A method for detecting a cancer which comprises

reacting a sample containing CEAs with a CEAs binding antibody and a specific sugar chain binding protein to give a complex I of CEAs and the CEAs binding antibody and a complex II of CEAs, the CEAs binding antibody and the specific sugar chain binding protein,

measuring an amount of the complex I and an amount of the complex II, and
using the amounts as an indicator for the detection.

(8) A method for detecting a cancer which comprises

reacting a sample containing CEAs with a CEAs binding antibody and a specific sugar chain binding protein to give a complex I of CEAs and the CEAs binding antibody and a complex II of CEAs, the CEAs binding antibody and the specific sugar chain binding protein,
measuring each independently an amount of the complex I and an amount of the complex II,

calculating a ratio of the amount of the complex II relative to a total amount of the complex I and complex II, and using the ratio as an indicator for the detection.

(9) A kit for detection of CEAs having a modified sugar chain structure which comprises a CEAs binding antibody and a specific sugar chain binding protein.

**BRIEF DESCRIRTION OF THE SEVERAL VIEWS OF THE DRAWINGS**

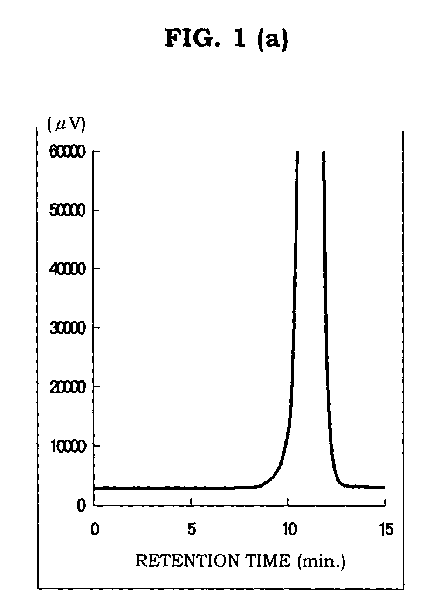

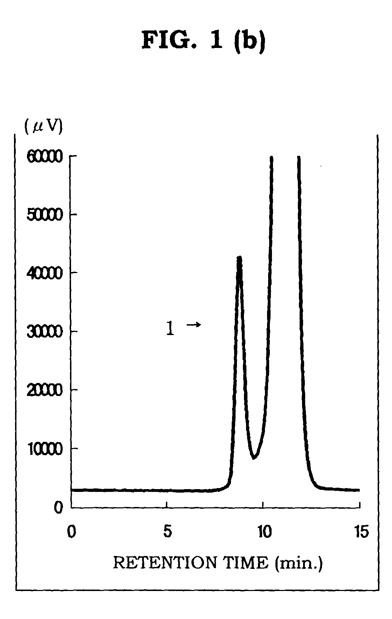

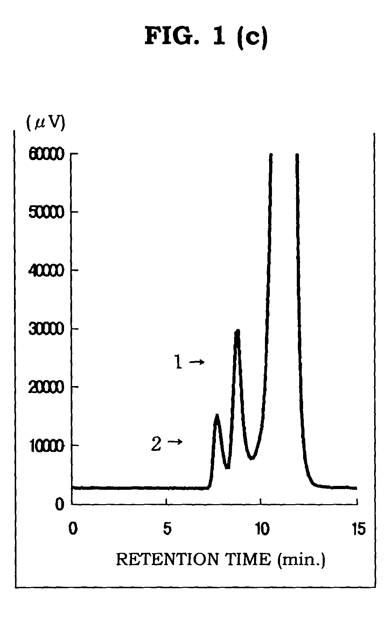

Fig. 1 shows an analysis pattern of a sample which was obtained by a high performance liquid chromatography (HPLC) in Example 1. Fig. 1 (a) is an elution pattern of a POD labeled CEAs binding antibody, Fig. 1 (b) is an elution pattern of a complex of CEAs and a POD labeled CEAs binding antibody (peak 1), and Fig. 1 (c) is an elution pattern of a complex of CEAs and a POD labeled CEAs binding antibody (peak 1) and a complex of CEAs, a POD labeled CEAs binding antibody and a specific sugar chain binding protein (antibody) (peak 2).

**DESCRIPTION OF THE PREFERRED EMBODIMENTS**

The CEAs binding antibody of the present invention is not particularly limited and any antibody may be used so long as it is an antibody capable of specifically binding to a constant region of CEAs. In the present invention, 'a constant region of CEAs" means a region common to all CEAs in body fluid (MASAHIDE KUROKI, HYBRIDOMA, 4 (11), 39 1-407 (1992)). In other words, the constant region is a region existed commnly in any of CEAs, regardless of their sugar chain structures or regardless of their origin, i.e. originated from normal cells or cancer cells. The antibody may be either of the following polyclonal antibody or monoclonal antibody: e.g. a polyclonal antibody prepared by immunizing animals such as a horse, a cattle, a sheep, a rabbit, a goat, a rat, a mouse, etc. with CEAs, according to a conventional method, for example, the method described in Matsuhashi Tadashi et al. "Menekijikkengaku Nyumon" 2nd ed., GAKKAI-SHUPPAN CENTERLtd., 1981, etc.; and a monoclonal antibody produced by hybridomas obtained by fusing cells from a tumor line of a mouse together with spleen cells derived from a mouse immunized with CEAs, according to a conventional method, i.e., a cell fusion method established by G. Kohler and C. Milstein (Nature, 256, 495, 1975). These antibodies may be used singly or in proper combination of two or more thereof.

The CEAs binding antibody includes an antibody having a property of binding to the constant region of CEAs but incapable of binding to CEAs having a modified sugar chain structure to which a protein capable of binding to a modified sugar chain structure (a specific sugar chain binding protein) is already bound (hereinafter abbreviated as a competitive CEAs binding antibody) and an antibody having a property of binding to all CEAs no matter whether a specific sugar chain binding protein is already bound or not (hereinafter abbreviated as a noncompetitive CEAs binding antibody).

The specific sugar chain binding protein in the present invention includes, for example, an antibody and a lectin, which are capable of specifically binding to a specific sugar chain structure of CEAs. Examples of the specific sugar chain binding protein include those recognizing a sugar chain containing a fucose residue and/or a sialic acid residue, more specifically, an anti-Lewis type sugar chain antibody capable of recognizing a Lewis type sugar chain structure such as an anti-Le$^a$ antibody, an anti-Le$^b$ antibody, an anti-Le$^x$ antibody and an anti-Le$^y$ antibody; an anti-sialyl Lewis type sugar chain antibody capable of recognizing a sialyl Lewis type sugar chain structure such as a A-Le$^a$ and a S-Le$^x$; a L-fucose binding lectin such as *Lotus tetragonolobus* agglutinin; a D-galactose or N-acetyl-D-galactosamine binding lectin such as *Arachis hypogaea* agglutinin, soybean agglutinin, *Ricinus communis* agglutinin and Phytohemagglutinin; a D-mannose binding lectin such as Concanavalin A, *Lens culinaris* agglutinin and *Pisum sativum* agglutinin; a N-acetylglucosamine binding lectin such as Wheat germ agglutinin and *Datura stramonium* agglutinin; a sialic acid binding lectin such as *Limulus polyphemus* agglutinin. Among them, a D-galactose or N-acetyl-D-galactosamine binding lectin and a D-mannose binding lectin are preferable. These specific sugar chain binding proteins may be used singly or in proper combination of two or more thereof.

In the above mentioned classification of lectins, "X binding lectin" means that a lectin which is once bound to a suitable sugar chain immobilized on an affinity column is easily eluted by "X". For example, "a D-galactose or N-acetyl-D-galactosamine binding lectin" means a lectin once bound to a suitable sugar chain immobilized on an affinity column is easily eluted by D-galactose or N-acetyl-D-galactosamine.

The antibody capable of recognizing a modified sugar chain structure of CEAs may be either of a polyclonal antibody or a monoclonal antibody prepared in accordance with the above mentioned conventional methods.

In the present invention, specific examples of the specific sugar chain are (1) those capable of binding to the above mentioned lectins, (2) those existing in the CEAs produced from a tumor cell such as colorectal cancer cells and the like. More specific examples are those described in Yamamoto, K., Eur. J. Biochem., 143 (1), 133-144, 1984, etc.

A method for detection of CEAs having a modified sugar chain structure of the present invention is conduced by using of a CEAs binding antibody and a specific sugar chain binding protein in a proper combination, and measuring an amount of a complex of CEAs, the CEAs binding antibody and the specific sugar chain binding protein or confirming no such complex produced.

A method for detecting a cancer of the present invention is conducted by detecting a cancer on the basis of an amount of CEAs having a modified sugar chain structure or an amount of other CEAs having a sugar chain structure other than the modified sugar chain structure, in other words, detecting a cancer using the amounts as an indicator for the detection. Specific examples of the methods are (1) a method comprising using an amount of a complex of CEAs, a CEAs binding antibody and a specific sugar chain binding protein measured by the above mentioned method as an indicator for the detection, (2) a method comprising using an amount of a complex II of CEAs (CEAs having a modified sugar chain structure), a CEAs binding antibody and a specific sugar chain binding protein and an amount of a complex I of CEAs (CEAs having a sugar chain structure other than the modified sugar chain structure) and the CEAs binding antibody as an indicator for the detection, and (3) a method comprising measuring an amount of a complex I of CEAs (CEAs having a sugar chain structure other than the modified sugar chain structure) and a CEAs binding antibody and an amount of a complex II of CEAs (CEAs having a modified sugar chain structure), the CEAs binding antibody and a specific sugar chain binding protein, calculating a ratio of the amount of the complex I or complex II relative to the total amount of these complexes (complex I and complex II), in other words, calculating a ratio of CEAs forming the complexes together with a CEAs binding antibody and a specific sugar chain binding protein, and using the ratio as an indicator for the detection.

Namely, in the present invention, specific examples of the concrete analyte(s) to be measured are total CEAs, CEAs having a modified sugar chain structure, CEAs having a sugar chain structure other than the modified sugar chain structure and the like. CEAs are decomposed into various fragments in a living body, and these fragments are also included in the analyte to be measured of the present invention so long as it is one capable of binding to a CEAs binding antibody and/or a specific sugar chain binding protein. These analytes may be measured separately or simultaneously in one shot.

In other words, a ratio of an amount of CEAs having a specific modified sugar chain structure or CEAs having a sugar chain structure other than the specific one relative to the total amount of CEAs, in a sample to be tested, is obtained by using a CEAs binding antibody and a specific sugar chain binding protein such as a lectin and an antibody mentioned above, and this ratio is compared with a ratio obtained by using a normal sample, whereby existence or non-existence of a cancer in the sample can be confirmed. For instance, when certain amount of the CEAs reacted to a specific sugar chain binding protein (i.e. the CEAs having a modified sugar chain structure) is found in a sample originated from a patient suffering from cancer, while no such CEAs is found in a sample originated from healthy human beings or the amount in the former is lager than that in the latter, on the basis of these differences, existence of a cancer in the sample can be confirmed. The amounts and the ratio mentioned above can be used also as an indicator for detecting a precancerous states of tissues or organs in human being, i.e. non-normal or abnormal states or symptoms of tissues or organs which are likely made into a cancer. Namely, the amount of the CEAs reacted to a specific sugar chain binding protein (i.e. the CEAs having a modified sugar chain structure) even in a precancerous states or symptoms of tissues or organs which are not yet in the state of a cancer is different from that in normal state. Thus "detection" of this kind of precancerous state or symptom is also included in the concept or definition of "detection of a cancer" in the present invention. The present invention has been completed on the basis of the above which has been found for the first time by the present inventors.

Specific examples of a method for measuring of CEAs are as follows.

## I. a method for each independently measuring analyte(s) to be measured

Analyte(s), namely, for example, total CEAs, CEAs having a specific modified sugar chain structure, CEAs having a sugar chain structure other than the specific one and the like can be measured respectively as follows.

## I-1. a measurement of the total CEAs

The measurement can be conducted according to a known method using a CEAs binding antibody.

## I-2. measurement of CEAs having a specific modified sugar chain structure

## I-2-1) a method using an insoluble carrier containing a CEAs binding antibody immobilized thereon

First, a sample derived from a living body such as plasma, serum, cerebrospinal fluid, various extraction solutions from body tissues, feces, and urine is reacted with a CEAs binding antibody immobilized on an insoluble carrier to form the following immobilized complex.

an insoluble carrier -[ a CEAs binding antibody ]-[ CEAs ]

And then, after removing unnecessary coexisting substances by washing, the said immobilized complex is reacted with a specific sugar chain binding protein having a labeling substance bound thereto (hereinafter abbreviated as a labeled specific sugar chain binding protein) to form the following immobilized complex.

an insoluble carrier -[ a CEAs binding antibody ]-[ CEAs ]-[ a labeled specific sugar chain binding protein ]

After removing the free labeled specific sugar chain binding protein by washing the said immobilized complex, an amount of the labeling substance in the immobilized complex is measured by a suitable method, and an amount of CEAs having a specific modified sugar chain structure in the sample can be obtained by applying the obtained measurement value to a calibration curve showing the relationship between the labeling substance (measurement value) and the concentration of CEAs which has been previously obtained by carrying out the same measurement as described above except for using standard solutions containing known concentrations of CEAs having the specific modified sugar chain structure.

**I-2-2) a method using an insoluble carrier containing a specific sugar chain binding protein immobilized thereon**

First, a sample derived from a living body such as plasma, serum, cerebrospinal fluid, various extraction solutions from body tissues, feces, and urine is reacted with a specific sugar chain binding protein immobilized on an insoluble carrier to form the following immobilized complex.

| an insoluble carrier |-[ a specific sugar chain binding protein ]-[ CEAs ]

And then, after removing unnecessary coexisting substances by washing, the said immobilized complex is reacted with a CEAs binding antibody having a labeling substance bound thereto (hereinafter abbreviated as a labeled CEAs binding antibody) to form the following immobilized complex.

| an insoluble carrier |-[ a specific sugar chain binding protein ]-[ CEAs ]-[ a labeled CEAs binding antibody ]

After removing the free labeled CEAs binding antibody by washing the said immobilized complex, an amount of the labeling substance in the immobilized complex is measured by a suitable method, and an amount of CEAs having a specific modified sugar chain structure in the sample can be obtained by applying the obtained measurement value to a calibration curve showing the relationship between the labeling substance (measurement value) and a concentration of CEAs which has been previously obtained by carrying out the same measurement as described above except for using standard solutions containing known concentrations of CEAs having the specific modified sugar chain structure.

**I-2-3) a method using a labeled specific sugar chain binding protein and a high performance liquid chromatography (HPLC)**

First, a sample derived from a living body such as plasma, serum, cerebrospinal fluid, various extraction solutions from body tissues, feces, and urine is reacted with a labeled specific sugar chain binding protein and a noncompetitive CEAs binding antibody to form the following immobilized complex.

[ a labeled specific sugar chain binding protein ]-[ CEAs ]-[ a noncompetitive CEAs binding antibody ]

And then, the said complex is separated from the free labeled specific sugar chain binding protein by using HPLC packed with a suitable carrier or an electrophoresis method, and an amount of the labeling substance in the complex is measured by a suitable method.
An amount of CEAs having a specific modified sugar chain structure in the sample can be obtained by applying the obtained measurement value to a calibration curve showing the relationship between the labeling substance (measurement value) and a concentration of CEAs which has been previously obtained by carrying out the same measurement as described above except for using standard solutions containing known concentrations of CEAs having the specific modified sugar chain structure.

**I-3. a measurement of CEAs having a sugar chain structure other than the specific one**

**I-3-1) a method using a free specific sugar chain binding protein**

First, a sample derived from a living body such as plasma, serum, cerebrospinal fluid, various extraction solutions from body tissues, feces, and urine is reacted with a specific sugar chain binding protein to produce a complex of CEAs having a specific modified sugar chain structure and a specific sugar chain binding protein (hereinafter, if necessary, abbreviated as a binding protein bound CEAs).

And then, a sample containing only a CEAs having a sugar chain structure other than the specific one (hereinafter, if necessary, abbreviated as a nonbound CEAs) is obtained by separating the binding protein bound CEAs and the CEAs having no specific sugar chain binding protein bound thereto, in other words, a nonbound CEAs from the sample by using a known separating method such as a centrifuge method, a gel filtration method, a molecular fractionation membrane method and an electrophoresis method.

An amount of nonbound CEAs can be measured by measuring CEAs in the sample obtained above which contains only a nonbound CEAs by a known method using a CEAs binding antibody.

The sample containing only a nonbound CEAs may be prepared by treating the sample derived from a living body with an affinity chromatography using a carrier to which a specific sugar chain binding protein is bound.

### I-3-2) a method using a free specific sugar chain binding protein

First, a CEAs binding antibody immobilized on an insoluble carrier is reacted with a sample derived from a living body such as plasma, serum, cerebrospinal fluid, various extraction solutions from body tissues, feces, and urine to form the following immobilized complex.

an insoluble carrier -[ CEAs binding antibody ]-[ CEAs ]

And then, after removing unnecessary coexisting substances by washing, the said immobilized complex is reacted with a specific sugar chain binding protein, and further reacted with a competitive CEAs binding antibody having a labeling substance bound thereto (hereinafter abbreviated as a labeled competitive CEAs binding antibody) to form the following immobilized complexes.

an insoluble carrier -[ CEAs binding antibody ]-[ CEAs ]-[ a specific sugar chain binding protein ]

an insoluble carrier -[ CEAs binding antibody ]-[ CEAs ]-[ a labeled competitive CEAs binding antibody ]

After removing the free labeled competitive CEAs binding antibody by washing the said immobilized complexes, an amount of the labeling substance in the immobilized complexes is measured by a suitable method, and an amount of nonbound CEAs in the sample can be obtained by applying the obtained measurement value to a calibration curve showing the relationship between the labeling substance (measurement value) and a concentration of CEAs which has been previously obtained by carrying out the same measurement as described above except for using standard solutions containing known concentrations of CEAs having a sugar chain structure other than the specific one, i.e., nonbound CEAs.

### I-3-3) a method using an insoluble carrier having a competitive CEAs binding antibody immobilized thereon

First, a sample derived from a living body such as plasma, serum, cerebrospinal fluid, various extraction solutions from body tissues, feces, and urine is reacted with a specific sugar chain binding protein to produce a binding protein bound CEAs, and this sample is reacted with a competitive CEAs binding antibody immobilized on an insoluble carrier to form the following immobilized complex.

an insoluble carrier -[ a competitive CEAs binding antibody ]-[ a nonbound CEAs ]

7

And then, after removing unnecessary coexisting substances by washing, the said immobilized complex is reacted with a labeled CEAs binding antibody to form the following immobilized complex.

| an insoluble carrier |-[ a competitive CEAs binding antibody ]-[ a nonbound CEAs ]-[ a labeled CEAs binding antibody ]

After removing the free labeled CEAs binding antibody by washing the said immobilized complex, an amount of the labeling substance in the immobilized complex is measured by a suitable method, and an amount of nonbound CEAs in the sample can be obtained by applying the obtained measurement value to a calibration curve showing the relationship between the labeling substance (measurement value) and a concentration of CEAs which has been previously obtained by carrying out the same measurement as described above except for using standard solutions containing known concentrations of nonbound CEAs.

### I-3-4) a method using a labeled competitive CEAs binding antibody and HPLC

First, a sample derived from a living body such as plasma, serum, cerebrospinal fluid, various extraction solutions from body tissues, feces, and urine is reacted with a specific sugar chain binding protein to produce a binding protein bound CEAs, and this sample is reacted with a labeled competitive CEAs binding antibody to form the following complex.

**[ a nonbound CEAs ]-[ a labeled competitive CEAs binding antibody]**

And then, the said complex is separated from the free labeled competitive CEAs binding antibody by using HPLC packed with a suitable carrier or an electrophoresis method, and an amount of the labeling substance in the complex is measured by a suitable method.

An amount of nonbound CEAs in the sample can be obtained by applying the obtained measurement value to a calibration curve showing the relationship between the labeling substance (measurement value) and a concentration of CEAs which has been previously obtained by carrying out the same measurement as described above except for using standard solutions containing known concentration of nonbound CEAs.

In the above mentioned method, an amount of CEAs having a modified sugar chain structure (a binding protein bound CEAs) can also be obtained by subtracting the amount of CEAs having a sugar chain structure other than the specific one (a nonbound CEAs) from the amount of the total CEAs, and the amount of CEAs having a sugar chain structure other than the specific one (a nonbound CEAs) can be obtained by subtracting the amount of CEAs having a specific modified sugar chain structure (a binding protein bound CEAs) from the amount of the total CEAs.

### II. a method for measuring analyte(s) to be measured in one shot

### II-1. a method using a labeled CEAs binding antibody and a specific sugar chain binding protein

The measurement can be conducted as follows according to the method described in JP-A 7-191027 using a CEAs binding antibody.

Namely, first, a sample derived from a living body such as plasma, serum, cerebrospinal fluid, various extraction solutions from body tissues, feces, and urine is reacted with a labeled CEAs binding antibody and a specific sugar chain binding protein to produce the following complexes.

**[a labeled CEAs binding antibody ]-[ CEAs ]**

**[ a labeled CEAs binding antibody ]-[ CEAs ]-[ a specific sugar chain binding protein]**

And then, these complexes and a free labeled CEAs binding antibody are each independently separated from the sample by using HPLC packed with a suitable carrier or an electrophoresis method, and an amount of the labeling substance in the each complexes is measured by a suitable method.

Amounts of CEAs having a specific modified sugar chain structure and CEAs having a sugar chain structure other than the specific one, and the total amount of these CEAs, i.e., all CEAs can be obtained in one shot by applying the obtained measurement values to a calibration curve showing the relationship between the labeling substance (measurement value) and a concentrations of various CEAs which has been previously obtained by carrying out the same measurement as described above expect for using standard solutions containing known CEAs having a specific modified sugar chain structure and/or CEAs having a sugar chain structure other than the specific one.

As the method for separating the complexes and free labeled CEAs binding antibody, a method using a HPLC is preferable, because it is easy to operate and possible to use repeatedly.

And as the CEAs binding antibody to be used, a noncompetitive one is preferable.

**II-2. a method using a noncompetitive CEAs binding antibody, a competitive CEAs binding antibody and a specific sugar chain binding protein**

First, a sample derived from a living body such as plasma, serum, cerebrospinal fluid, various extraction solutions from body tissues, feces, and urine is reacted with a noncompetitive CEAs binding antibody having a suitable labeling substance bound thereto (hereinafter abbreviated as a labeled noncompetitive CEAs binding antibody), a competitive CEAs binding antibody and a specific sugar chain binding protein to form the following complexes.

**[ a labeled noncompetitive CEAs binding antibody ]-[ CEAs ]-[ a competitive CEAs binding antibody]**

**[ a labeled noncompetitive CEAs binding antibody ]-[ CEAs ]-[ a specific sugar chain binding protein ]**

And then, these complexes and the free labeled noncompetitive CEAs binding antibody are each independently separated from the sample by using HPLC packed with a suitable carrier or an electrophoresis method, and an amount of the labeling substance in each of the complexes is measured by a suitable method.

CEAs having a specific modified sugar chain structure and CEAs having a sugar chain structure other than the specific one, and the total amount of these CEAs, i.e., all CEAs can be obtained in one shot by applying the obtained measurement values to a calibration curve showing the relationship between the labeling substance (measurement value) and a concentrations of various CEAs which has been previously obtained by carrying out the same measurement as described above except for using standard solutions containing known CEAs having a specific modified sugar chain structure and/or CEAs having a sugar chain structure other than the specific one.

As the method for separating the complexes and the free labeled noncompetitive CEAs binding antibody, a method using a HPLC is preferable, because it is easy to operate and possible to use repeatedly.

**II-3. a method using a labeled CEAs binding antibody, a competitive CEAs binding antibody and a specific sugar chain binding protein**

First, a sample derived from a living body such as plasma, serum, cerebrospinal fluid, various extraction solutions from body tissues, feces, and urine is reacted with a labeled CEAs binding antibody, and after the reaction, the reaction solution is further reacted with a specific sugar chain binding protein and a competitive CEAs binding antibody to form the following complexes.

**[ a labeled CEAs binding antibody ]-[ CEAs ]-[ a competitive CEAs binding antibody ]**

**[ a labeled CEAs binding antibody ]-[ CEAs ]-[ a specific sugar chain binding protein ]**

And then, these complexes and the free labeled CEAs binding antibody are each independently separated from the sample by using HPLC packed with a suitable carrier or an electrophoresis method, and an amount of the labeling substance in each of the complexes is measured by a suitable method.

CEAs having a specific modified sugar chain structure and CEAs having a sugar chain structure other than the specific one, and the total amount of these CEAs, i.e., all CEAs can be obtained in one shot by applying the obtained measurement values to a calibration curve showing the relationship between the labeling substance (measurement value) and a concentrations of various CEAs which has been previously obtained by carrying out the same measurement as described above except for using standard solutions containing known CEAs having a specific modified sugar chain structure and/or CEAs having a sugar chain structure other than the specific one.

Further, in the above mentioned method, it is preferable to react the reaction product of the labeled CEAs binding antibody, after the reaction, further with a CEAs binding antibody having an epitope different from that of a labeled CEAs binding antibody. According to the mentioned above, it is possible to make larger the difference between the property of the complex and that of the ingredients in the serum which influence upon the measurement, whereby the influence by the ingredients in the serum can be lowered, and thus more accurate measurement can be attained.

As the method for separating the complexes and the free labeled noncompetitive CEAs binding antibody, a method using a HPLC is preferable because it is easy to operate and possible to use repeatedly.

The known method for measuring CEAs using a CEAs binding antibody can be conducted by using, for example, a CEAs binding antibody as a anti-CEAs antibody, according to an immunological method such as so-called enzyme immunoassay (EIA), radio immunoassay (RIA), enzyme-linked immunosorbent assay (ELISA) and a method using a HPLC described in JP-A 9-301995 etc.

The measurement principle may be any of a Sandwich method, a competitive method, a double antibody method, etc.

As the insoluble carrier used to immobilize the various CEAs binding antibody or the specific sugar chain binding protein, there can be exemplified by the following ones which have usually been used in the field of the above mentioned immunological method: e.g. an insoluble carrier such as a bead, a tube, a tray formed with many tubes and a microtiterplate, which are prepared from a metal, a glass, a ceramic, a silicone rubber and synthetic polymers such as polystylene, polyvinyl chloride, polypropylene, acryl polymers and polymethylacrylate. As the method for immobilizing the various CEAs binding antibody or the specific sugar chain binding protein to the insoluble carrier, there can be exemplified by methods usually used in the field of the above mentioned immunological method such as a physical absorption method and a chemical absorption method.

As the labeling substance to be bound to the CEAs binding antibody or the specific sugar chain binding protein in the present invention includes, for example, enzymes such as alkaline phosphatases, $\beta$-galactosidase, peroxidase (POD), micro-peroxidase, glucose oxidase, glucose-6-phosphate dehydrogenase, acetylcholine esterase, malate dehydrogenase and luciferase, which are used, for example, in enzyme immunoassay (EIA); radioisotopes such as $^{99m}$Tc, $^{131}$I, $^{125}$I, $^{14}$C, and $^{3}$H, which have been used, for example, in radioimmunoassay (RIA); fluorescence substances such as fluorescein, dancyl, fluorescamine, cumarin, europium, naphthylamine and their derivatives, which have been used, for example, in fluoroimmunoassay (FIA); luminescent substances such as luciferin, isoluminol, luminol and bis(2,4,6-triflorophenyl) oxalate; substances having absorption in UV-ray region, such as phenol, naphthol, anthracene and their derivatives; and substances having spin-labeling properties, which are represented by compounds having an oxyl group, such as 4-amino-2,2,6,6-tetramethylpiperidin- 1 -oxyl, 3-amino-2 ,2 , 5, 5-tetramethylpyrrolidin- 1-oxyl and 2, 6-di-t-butyl- $\alpha$ -(3, 5-di-t-butyl-4-oxo-2 ,5-cyclohexadien- 1-ylidene)-p-tolyloxyl. As the method for binding the above mentioned labeling substances to the various CEAs binding antibody or the specific sugar chain binding protein, (i.e. a method for labeling the above mentioned labeling substances to the various CEAs binding antibody or the specific sugar chain binding protein), there can be exemplified by a known standard method usually used, for example, in conventional EIA, RIA and FIA.

As the labeling method, use may be made of a known method using a reaction of avidin (or streptoavidin) with biotin.

As an apparatus to be used in the method of the present invention for using HPLC, there can be exemplified by one usually used in this field.

In the method of using HPLC of the present invention, in order to separate completely the complex from the free labeled CEAs binding antibody (or the free labeled specific sugar chain binding protein), the CEAs binding antibody or the specific sugar chain binding protein to which a substance to improve the separation effect (hereinafter abbreviated as a separation-improving substance) is bound can be used.

The separation-improving substance to be used for such purpose includes, for example, proteins such as $\alpha$ -chymotrypsinogen, $\beta$ - galactosidase, lysozyme, cytochrome C and trypsin inhibitors; peptides containing aminoacids such as phenylalanine, proline, arginine, lysin, aspartic acid and glutamic acid; halogen atoms such as bromine, chlorine and iodine; synthetic polymers such as polyethylene glycol; poly(amino acid)s such as poly(glutamic acid)s, poly(aspartic acid)s, polylysines, polyarginines, polyphenylalanines and polythyrosines; compounds containing alkyl chains having 3 to 10 carbon atoms such as fatty acids (e.g. palmitic acid, oleic acid, stearic acid, etc.); chemical substances which have a reactive group capable of binding to a CEAs binding antibody or a specific sugar chain binding protein and have hydrophobic or ionic properties such as N-( $\epsilon$ - maleimidocaproyloxy) succinimide (hereinafter abbreviated as EMCS), N-succinimidyl-6-maleimidohexanoate, bismaleimidohexane (hereinafter abbreviated as BMH) and octylamine; peptides containing a strong acid residue described in JP-A 9-301995 such as 4-(p-maleimidophenyl)butyryl-alanine- (tyrosine($SO_3H$))$_5$ and 4-(p-maleimidophenyl)butyryl-alanine- (tyrosine($SO_3H$))$_8$. The separation-improving substance may be used by properly considering properties (e.g. pH stability, hydrophobicity, solubility in an aqueous solution, isoelectric point, etc.) of CEAs, the CEAs binding antibody and the specific sugar chain binding proteins as the analyte(s) to be measured.

The method for binding the separation-improving substance to the CEAs binding antibody and/or the specific sugar chain binding protein can be conducted according to a known method such as (1) a known method for binding the labeling substance to the antibody usually used in a known EIA, RIA and FIA (e.g. Yuichi Yamamura "Igaku Jikken Koza Vol. 8" 1st ed.,NAKAYAMA-SHOTEN Ltd., 1971; Akira Kawano "Zusetsu Keikokotai" 1st ed., Soft Science, Inc., 1983; and Eiji Ishikawa, Tadashi Kawai and Kiyoshi Miyai "Koso Men-eki Sokuteiho" 2nd ed., IGAKU-SHOIN Ltd., 982), (2) a known method for modifying and binding a substance (e.g. Ikuzo Uritani, Kensuke Shimura, Michinori Nakamura and Masaru Funazu "Chemical Modification of Proteins Vols 1 and 2" 1st ed., GAKKAI-SHUPPAN CENTER Ltd., 1981; Yuji Inada et al. "Polyethylene Glycol-Modified Proteins" Seikagaku Vol. 62, No. 11, pp. 1351-1362, Japanese Biochemical Association, 1990; and Georg H. K. and Mark M. M. "DNA PROBES" STOCKTON PRESS, 1989).

The detection of a cancer (e.g. detection of existence or confirmation of non-existence of cancer cells) in a sample can be conducted by using, in proper combination, the total amount of CEAs, an amount of the CEAs having a specific modified sugar chain structure and an amount of the CEAs having a sugar chain structure other than the specific one, which are obtained by the present invention.

And further, a kind of a cancer and a region of existence of a cancer can also be determined by conducting the measurement with the use of plural kinds of the specific sugar chain binding proteins and analyzing the results.

The kit for detection of CEAs of the present invention is used for the above mentioned method of the present invention and comprises the CEAs binding antibody and the specific sugar chain binding protein. Preferable properties and specific examples of the main constituents of the kit are as described above.

The kit may contain reagents usually used in the art, such as buffers, reaction accelerators, sugars, proteins, salts, stabilizers (e.g. surfactants) and antiseptics, so long as they do not decrease the stability of reagents and do not inhibit the reaction of CEAs, the CEAs binding antibody and/or the specific sugar chain binding protein. The concentrations of the reagents may be properly chosen from ranges usually employed in the art.

The kit may contain metal ions such as magnesium ions which have been well known to affect the activity and stability of a lectin.

As the buffers usable in the reagent or kit of the present invention, there can be exemplified by all buffers usually used in immunotubidimetry, immunonephelometry, RIA and EIA, such as Tris buffers, phosphate buffers, veronal buffers, borate buffers and Good's buffers. The pH upon the reaction for measurement is not particularly limited so long as it dose not inhibit the antigen-antibody reaction and the reaction of the CEAs with the lectin.

The present invention is explained below in further detail with reference to Examples, which are not by way of limitaion but by way of illustration.

**EXAMPLES**

**Example 1** Method for separately measuring the sugar chains of CEAs

**[POD labeled CEAs binding antibody]**

Anti-CEA antibody (available from Wako Pure Chemical Industries, Ltd.) was used as a CEAs binding antibody. This antibody was made into Fab' by a conventional method, and this Fab' was bound to POD by a conventional method, whereby a POD labeled CEAs binding antibody was obtained.

**[Sample]**

CEA (available from COSMO-Bio, Ltd.) was dissolved in 50 mM phosphate buffer (pH 6.5) containing 0.9 % NaCl and 1 % BSA to prepare a solution of a concentration of 1,000 ng/ml. The solution was used as a sample.

**[Apparatus for analyzing and reagents for separately measuring the sugar chains of CEAs]**

The following apparatus and reagents were used.

**[HPLC conditions]**

Apparatus for analyzing:
HPLC (LC-9A, available from Shimazu Corporation.)
Column for analyzing:
Diol-300, 8.0 mm in diameter × 300 mm (available from Wako Pure Chemical Industries, Ltd.), a molecular weight by gel filtration of 22,000 to 660,000
Eluent for analyzing:
50 mM PBS pH 7.5
Substrate solution for analyzing:
25 mM acetoamidophenol (15 mM citric acid buffer, pH 5.5)
Flow rate:

eluent 1 ml/min., substrate solution 0.1 ml/min.

Detection:

Ex 328 nm, Em 432 nm

**[Analyzing procedure]**

(1) Confirmation of an eluting position of the CEAs binding antibody

To 30 µl of pure water was added 30 µl of the POD labeled CEAs binding antibody solution (a concentration of antibody: $1 \times 10^{-8}$ M), followed by incubating at 30 °C for 30 minutes. And then, 30 µl of thus obtained mixture was passed through a gel filtration column, and the substrate solution for the analysis was added to the effluent on-line to conduct a reaction at 60 °C for 30 seconds, and an amount of fluorescence produced by the reaction was detected.

(2) Confirmation of an eluting position of total CEAs

To 30 µl of the sample was added 30 µl of the POD labeled CEAs binding antibody solution (a concentration of antibody: $1 \times 10^{-8}$ M), followed by allowing a reaction to take place at 30 °C for 30 minutes. And then, 30 µl of thus obtained reaction solution was passed through a gel filtration column, and the substrate solution for the analysis was added to the effluent on-line to conduct a reaction at 60 °C for 30 seconds, and an amount of fluorescence produced by the reaction was detected.

(3) Confirmation of an eluting position of total CEAs having a specific modified sugar chain structure

To 30 µl of the sample was added 30 µl of the POD labeled CEAs binding antibody solution (a concentration of antibody: $1 \times 10^{-8}$ M), followed by allowing a reaction to take place at 30 °C for 30 minutes. And then, 30 µl of 50 mM phosphate buffer solution (pH 6.5, a concentration of antibody: $1 \times 10^{-7}$ M) containing an antibody against Le$^b$ which was a specific sugar chain binding protein (available from Signet corporation), 0.9 % NaCl and 1 % BSA was added to the reaction solution, followed by allowing a reaction to take place at 30 °C for 30 minutes. After the reaction, 30 µl of thus obtained reaction solution was passed through a gel filtration column and, the substrate solution for the analysis was added to the effluent on-line to conduct a reaction at 60 °C for 30 seconds, and an amount of fluorescence produced by the reaction was detected.

**[Results]**

Fig. 1 shows an analyzing pattern obtained by HPLC.

From the result of the Fig. 1 (a), it was found that the free POD labeled CEAs binding antibody was eluted at the position of retention time of about 11 minutes. On the other hand, when the POD labeled CEAs binding antibody was reacted with the sample, the peak 1 for a complex of the CEAs and the POD labeled CEAs binding antibody appeared at the position of retention time of about 8.5 minutes (Fig. 1(b)), and when the specific sugar chain binding antibody was added to the above reaction product to cause a rection, the peak 2 for a complex of the POD labeled CEAs binding antibody, CEAs and the specific sugar chain binding antibody appeared at the position of retention time of about 7.5 minutes (Fig. 1 (c)) When the same experiment mentioned above was also conducted by using an anti-Le$^y$ antibody in place of the anti-Le$^b$ antibody, appearance of the peak 2 for a complex of the POD labeled CEAs binding antibody, CEAs and the specific sugar chain binding antibody (peak 2) was confirmed.

From the above, by the method of the present invention using the CEAs binding antibody and the specific sugar chain binding protein, it was found that the CEAs having a specific modified sugar chain structure and the CEAs having a sugar chain structure other than the specific one can be separately detected.

**Example 2** Detection of a cancer by the method of the present invention

**[POD labeled CEAs binding antibody]**

A POD labeled CEAs binding antibody was prepared by the same manner as Example 1.

**[Samples]**

As samples, there were used nine sera originated from cancer patients and four sera originated from normal human beings (normal sera).

**[Method for separately measuring the various CEAs]**

Using the same apparatus and reagents as Example 1, the following was conducted. The HPLC conditions were the same as in Example 1.

**[Analyzing procedure]**

(1) To 30 µl of the sample was added 30 µl of the POD labeled CEAs binding antibody solution (a concentration of antibody: $1 \times 10^{-8}$ M), followed by allowing a raction to take place at 30 °C for 30 minutes. And then, 30 µl of 50 mM phosphate buffer solution (pH 6.5, a concentration of antibody: $1 \times 10^{-7}$ M) containing an antibody against any one of S-Le$^a$, S-Le$^x$, Le$^a$ and Le$^y$, which was a specific sugar chain protein (antibody) (an anti-S-Le$^a$ antibody and an anti-S-Le$^x$ antibody are available from Wako Pure Chemical Industries, Ltd., and an anti-Le$^a$ antibody and an anti-Le$^y$ antibody are available from Signet corporation), 0.9 % NaCl and 1 % BSA was added to the reaction solution, followed by allowing a reaction to take place at 30 °C for 30 minutes.
(2) After the reaction, 30 µl of thus obtained reaction solution was passed through a gel filtration column and, the substrate solution for the analysis was added to the effluent on-line to conduct a reaction at 60 °C for 30 seconds, and an amount of fluorescence produced by the reaction was detected.

[Concentration of CEAs and calculation method]

A ratio (%) of the amount of the CEAs having a specific modified sugar chain structure relative to the amount of total CEAs was calculated by applying the results obtained according to the above mentioned method to the following equation. In the equation, peak 1 was the peak for a complex of the CEAs and the POD labeled CEAs binding antibody, and peak 2 was the peak for a complex of the POD labeled CEAs binding antibody, CEAs and the specific sugar chain binding antibody.

**A ratio (%) of the amount of the CEAs having a specific modified sugar chain structure relative to the amount of total CEAs = [a peak area for the peak 2 / (a peak area for the peak 1 + a peak area for the peak 2)] × 100**

The results obtained were shown in Table 1.

Table 1

| Sample Origin | A ratio (%) of the amount of the CEAs reacted to a specific anti-sugar chain antibody (an amount of the CEAs having a specific modified sugar chain structure) relative to the amount of total CEAs | | | |
|---|---|---|---|---|
| | Anti-S-Le$^a$ antibody | Anti-S-Le$^x$ antibody | Anti-Le$^a$ antibody | Anti-Le$^y$ antibody |
| | % | % | % | % |
| Rectal cancer | 1.6 | - | 1.1 | - |
| Rectum cancer | - | - | - | 2.0 |
| Lung cancers | 2.3 | 3.1 | 2.2 | - |
| | - | - | 2.2 | - |
| Liver cancer | 5.9 | - | - | - |
| Oropharyngeal cancer | 17.7 | - | - | - |
| Breast cancer | - | 3.3 | - | 5.4 |

Table 1 (continued)

| Sample Origin | A ratio (%) of the amount of the CEAs reacted to a specific anti-sugar chain antibody (an amount of the CEAs having a specific modified sugar chain structure) relative to the amount of total CEAs | | | |
| --- | --- | --- | --- | --- |
| | Anti-S-Le$^a$ antibody | Anti-S-Le$^x$ antibody | Anti-Le$^a$ antibody | Anti-Le$^y$ antibody |
| | % | % | % | % |
| Cervix uteri cancer | 8.1 | 6.4 | - | - |
| Matastasis of bone marrow lymph node | - | 1.2 | - | - |
| Normal human beings | - | - | - | - |
| | - | - | - | - |
| | - | - | - | - |
| | - | - | - | - |
| -: not detected | | | | |

As is clear from Table 1, no CEAs having a specific modified sugar chain structure was detected from normal human beings. On the other hand, from sera originated from cancer patients, various CEAs having a modified sugar chain structure were detected, and it was confirmed that varieties of sugar chain structures differed among the kinds of cancers.

For example, in rectal cancer, S-Le$^a$ and Le$^a$ were detected, and in rectum cancer, Le$^y$; in lung cancer, S-Le$^a$, S-Le$^x$ and Le$^a$; in liver cancer and pharyngitis cancer, S-Le$^a$; in breast cancer, S-Le$^x$ and Le$^y$; in cervix uteri cancer, S-Le$^a$ and S-Le$^x$; in matastasis of bone marrow lymph node, S-Le$^x$ were detected respectively. Namely, it is found that the kinds of the modified sugar chain structures differ among the kinds of cancers.

From the above, it is suggested that the method for separately measuring the various CEAs can be used for detecting cancers and is extremely useful for defining the kinds of cancers.

The present invention provides an easy and simple method for separately measurement of CEAs having a modified sugar chain structure and a kit used in said method. By using, in proper combination, the measurement results on various CEAs obtained by the method of the present invention, existence or non-existence of cancers and the kinds of the cancers can be also detected.

## Claims

1. A method for detection of carcinoembryonic antigens having a modified sugar chain structure which comprises using an antibody against a constant region of carcinoembryonic antigens and a protein capable of recognizing a modified sugar chain structure of carcinoembryonic antigens.

2. A method for detection of carcinoembryonic antigens having a modified sugar chain structure which comprises measuring an amount of a complex of carcinoembryonic antigens, an antibody against a constant region of carcinoembryonic antigens and a protein capable of recognizing a modified sugar chain structure of carcinoembryonic antigens.

3. A method for detection of carcinoembryonic antigens having a modified sugar chain structure which comprises

   reacting a sample with an antibody against a constant region of carcinoembryonic antigens and a protein capable of recognizing a modified sugar chain structure of carcinoembryonic antigens to give a complex of carcinoembryonic antigens, the specific antibody and the protein, and
   measuring an amount of the complex.

4. A method according to any one of Claim 1 to 3, wherein the protein is an antibody or a lectin.

5. A method according to Claim 4, wherein the antibody is one recognizing a sugar chain containing a fucose residue and/or a sialic acid residue.

6. A method according to Claim 4, wherein the antibody is an anti-Lewis type sugar chain antibody or an anti-sialyl Lewis type sugar chain antibody.

7. A method according to Claim 6, wherein the anti-Lewis type sugar chain antibody is an anti-Le$^a$ antibody, an anti-Le$^b$ antibody, an anti-Le$^x$ antibody or an anti-Le$^y$ antibody.

8. A method according to Claim 6, wherein the anti-sialyl Lewis type sugar chain antibody is an anti-S-Le$^a$ antibody or an anti-S-Le$^x$ antibody.

9. A method according to Claim 4, wherein the lectin is an L-fucose binding lectin, a D-galactose or an N-acetyl-D-galactosamine binding lectin, a D-mannose binding lectin, an N-acetylglucosamine binding lectin or a sialic acid binding lectin.

10. A method according to Claim 4, wherein the lectin is Concanavalin A, *Ricinus communis* agglutinin, *Lens culinaris* agglutinin or Phytohemagglutinin.

11. A method for detecting a cancer which comprises using an amount of a carcinoembryonic antigens having a modified sugar chain structure as an indicator for the detection.

12. A method for detecting a cancer which comprises

measuring an amount of a complex of carcinoembryonic antigens, an antibody against a constant region of carcinoembryonic antigens and a protein capable of recognizing a modified sugar chain structure of carcinoembryonic antigens, and
using the amount as an indicator for the detection.

13. A method for detecting a cancer which comprises

measuring an amount of a complex of carcinoembryonic antigens, an antibody against a constant region of carcinoembryonic antigens and a protein capable of recognizing a modified sugar chain structure of carcinoembryonic antigens, and an amount of a complex of carcinoembryonic antigens and the antibody, and
using the amounts as an indicator for the detection.

14. A method for detecting a cancer which comprises

reacting a sample containing carcinoembryonic antigens with an antibody against a constant region of carcinoembryonic antigens and a protein capable of recognizing a modified sugar chain structure of carcinoembryonic antigens to give a complex I of carcinoembryonic antigens and the antibody and a complex II of carcinoembryonic antigens, the antibody and the protein,
measuring an amount of the complex I and an amount of the complex II, and
using the amounts as an indicator for the detection.

15. A method for detecting a cancer which comprises

reacting a sample containing carcinoembryonic antigens with an antibody against a constant region of carcinoembryonic antigens and a protein capable of recognizing a modified sugar chain structure of carcinoembryonic antigens to give a complex I of carcinoembryonic antigens and the antibody and a complex II of carcinoembryonic antigens, the antibody and the protein,
measuring each independently an amount of the complex I and an amount of the complex II,
calculating a ratio of the amount of the complex II relative to a total amount of the complex I and complex II, and
using the ratio as an indicator for the detection.

16. A method according to any one of Claim 12 to 15, wherein the protein is an antibody or a lectin.

17. A method according to Claim 16, wherein the antibody is one recognizing a sugar chain containing fucose residue and/or a sialic acid residue.

18. A method according to Claim 16, wherein the antibody is an anti-Lewis type sugar chain antibody or an anti-sialyl Lewis type sugar chain antibody.

19. A method according to Claim 18, wherein the anti-Lewis type sugar chain antibody is an anti-Le$^a$ antibody, an anti-Le$^b$ antibody, an anti-Le$^x$ antibody or an anti-Le$^y$ antibody.

20. A method according to Claim 18, wherein the anti-sialyl Lewis type sugar chain antibody is an anti-S-Le$^a$ antibody or an anti-S-Le$^x$ antibody.

21. A method according to Claim 16, wherein the lectin is an L-fucose binding lectin, a D-galactose or an N-acetyl-D-galactosamine binding lectin, a D-mannose binding lectin, an N-acetylglucosamine binding lectin or a sialic acid binding lectin.

22. A method according to Claim 16, wherein the lectin is Concanavalin A, *Ricinus communis* agglutinin, *Lens culinaris* agglutinin or Phytohemagglutinin.

23. A kit for detection of carcinoembryonic antigens having a modified sugar chain structure which comprises an antibody against a constant region of carcinoembryonic antigens and a protein capable of recognizing a modified sugar chain structure of carcinoembryonic antigens.

24. A kit according to Claim 23, wherein the protein is an antibody or a lectin.

25. A kit according to Claim 24, wherein the antibody is one recognizing a sugar chain containing a fucose residue and/or a sialic acid residue.

26. A kit according to Claim 24, wherein the antibody is an anti-Lewis type sugar chain antibody or an anti- sialyl Lewis type sugar chain antibody.

27. A kit according to Claim 26, wherein the anti-Lewis type sugar chain antibody is an anti-Le$^a$ antibody, an anti-Le$^b$ antibody, an anti-Le$^x$ antibody or an anti-Le$^y$ antibody.

28. A kit according to Claim 26, wherein the anti-sialyl Lewis type sugar chain antibody is an anti-S-Le$^a$ antibody or an anti-S-Le$^x$ antibody.

29. A kit according to Claim 24, wherein the lectin is an L-fucose binding lectin, a D-galactose or an N-acetyl-D-galactosamine binding lectin, a D-mannose binding lectin, an N-acetylglucosamine binding lectin or a sialic acid binding lectin.

30. A kit according to Claim 24, wherein the lectin is Concanavalin A, *Ricinus communis* agglutinin, *Lens culinaris* agglutinin or Phytohemagglutinin.

# FIG. 1 (a)

# FIG. 1 (b)

# FIG. 1 (c)